# EUROPEAN PATENT SPECIFICATION

(11) **EP 1 173 217 B1**
(45) Date of publication and mention of the grant of the patent: **19.10.2005**
(21) Application number: 00927515.7
(22) Date of filing: 05.05.2000
(51) Int. Cl.: A61K 47/12, A61K 47/14, A61K 47/44, A61K 48/00

(54) **ENHANCED DELIVERY OF NUCLEIC ACID-BASED DRUGS**
GESTEIGERTE ABGABE VON AUF NUKLEINSÄUREN BASIERENDEN ARZNEIMITTELN
APPORT AMELIORE DE MEDICAMENTS A BASE D'ACIDE NUCLEIQUE

(30) Priority: 05.05.1999 US 132603 P
(43) Date of publication of application: 23.01.2002
(73) Proprietor: Merrion Research I Limited, Dublin 24 (IE)
(72) Inventor: SEVESO, Michela, Dublin 1 (IE); O'MAHONY, Daniel, J., Blackrock County, Dublin (IE); PAGE, David, T., Clondalkin, Dublin 22 (IE)
(74) Representative: Denison, Christopher Marcus
(86) International application number: PCT/GB2000/001726
(87) International publication number: WO 2000/067798

(56) References cited:
- EP-A- 0 890 362
- WO-A-00/02601
- WO-A-95/26718
- WO-A-99/01579
- WO-A-99/60167

## Description

### FIELD OF THE INVENTION

The present invention relates to the use of permeation enhancers, particularly paracellular and transcellular permeation enhancers, to improve the intracellular delivery to mammalian cells of nucleic acid-based drugs such as antisense oligonucleotides and genes. In particular, this invention relates to formulations which contain a nucleic acid-based drug in combination with at least one permeation enhancer, such as caprylic acid, capric acid or their pharmaceutically acceptable salts, to facilitate the intracellular delivery of the nucleic acid-based drug.

### BACKGROUND OF THE INVENTION

Several issues are associated with the delivery of nucleic acid-based drugs, such as antisense oligonucleotides and genes, into cells. Cellular delivery of oligonucleotides has been a challenge and it has been reported that only 1-2% of oligonucleotides are' taken up by cells (1988, Gene 72, 333-341; 1989, Proc. *Natl. Acad. Sci. U.S.A.* 86, 3474-3478). Cell penetration, release from endosomal compartments and intracellular distribution of antisense oligonucleotides are among the major limiting factors for the effective use of antisense oligonucleotides as drugs. Because cell membranes are negatively charged, the passive diffusion of phosphorothioate-based antisense oligonucleotides across the lipid bilayer is a major challenge. Furthermore, oligonucleotide length, chemistry , concentration and sequence have been shown to be determinants of cellular uptake. Cell type, stage of cell cycle, degree of cell differentiation have also been proposed to affect uptake levels (1998, *J. Drug Targeting* 5, 225-234; 1995, *J. Pharmacal. Toxicol*. 275, 462).

Several mechanisms by which oligonucleotides might be internalised have been identified. The internalisation process is time and temperature dependent and saturable, suggesting that an active/energy-dependent mechanism is involved. Receptor-mediated endocytosis of oligonucleotide sequences has been observed in a number of cell types. For instance, proteins of 80 and 30kDa have been identified that mediate oligonucleotide uptake, whereas a simple charge association with the membrane has been found to be sufficient co trigger endo- or pinocytosis (1989, *Proc. Natl. Acad. Sci. U*.*S*.*A*. 86, 6454-6458; 1985, *J. Clin. Invest*. 76, 2182-2190).

Once inside the cell, oligonucleotides have been shown to partition to various regions of the cell, depending on their chemistry. For example phosphodiescer- and methylphosphonate-based oligonucleotides are sequestered within the nucleus in most of the cell types examined (1990, *New Biol*. 9, 1091-1100). Phosphorothioate derivatives remain within the cytoplasm in some cell lines, but enter the nucleus in others (1996, Trends *Biotechnology* 14(5), 147-9).

WO99/01579 discloses that certain permeation enhancers are able to increase uptake of nucleic acids from the alimentary canal into the bloodstream.

WO 99/60167 discloses that certain permeability enhancers are able to increase delivery of nucleic acids through the skin. EP 0 890 362 and WO 95/26718 describe the use of permeability enhancers to aid delivery of nucleic acids into cells. The enhances in EP 0 890 362 are a probe polar compounds.

Despite these efforts and others, the intracellular delivery, especially via the oral route, of pharmacologically effective amounts of nucleic acid-based drugs remains problematic.

### SUMMARY OF THE INVENTION

It has now been discovered that enhancers such as sodium caprylate and sodium caprate can be used to achieve elevated intracellular delivery of a nucleic acid-based drug to mammalian cells. In particular, the enhancers are capable of increasing delivery of nucleic acid-based drugs to the cytoplasm and/or nucleus of cells, compared to the delivery to these regions in the absence of enhancer. In the absence of enhancer, uptake of nucleic acid, if any, tends to be weak and into intracellular vesicles such as endosomes rather than into the cytoplasm or nucleus.

The enhanced delivery of the invention can lead to increased biological activity *in vitro* and *in vivo,* such as substantially decreased expression of a particular receptor upon administration in the presence of an enhancer of an antisense oligonucleotide to that receptor or increased efficiency of DNA transfection upon delivery of plasmid DNA, condensed plasmid DNA or plasmid DNA associated with cationic lipid(s) or cationic polymer(s) in combination with an enhancer in comparison to DNA delivered in the absence of the enhancer.

Thus, this invention provides methods and associated uses as defined in the appended claims for enhancing the intracellular delivery of a nucleic acid-based drug, such as an oligonucleotide or gene, that comprise the administration of the nucleic acid-based drug in combination or sequentially with an enhancer. The enhancer is present in an amount effective to enhance the intracellular delivery of the nucleic acid-based drug. Preferred enhancers include caprylic acid, capric acid and their pharmaceutically acceptable salts. The enhancement is not by topical administration of the medicament.

Elevated intracellular delivery may be indicated by homogeneous, rather than punctate, distribution of the nucleic acid-based drug within the cell, particularly within the cytoplasm and/or nucleus, or by measuring activity of the drug, for example elevated expression of a protein encoded by the drug, or reduced expression in the case of an antisense drug. The distribution or activity may be determined by any convenient manner, such as those exemplified herein, or others well known to those skilled in the art.

### BRIEF DESCRIPTION OF THE DRAWINGS

**Fig. 1a** shows the cell survival (% relative to untreated cells) for Caco-2 cells incubated for either 30 min or 150 min with C8 at concentrations of 120, 100, 80, 60,40, 20 and 10 mM using the MTT assay as described in Example 1, Experiment d. Fig. 1b shows the cell survival (% relative to untreated cells) for Caco-2 cell incubated for either 30 min or 150 min with C10 at concentrations of 14, 12, 10, 8, 6, 4, and 2 mM using the MTT assay as described in Example 1, Experiment d;
**Fig. 2** shows a Western blot analysis depicting the expression of HPT-1 receptor in Caco-2 non-differentiated cells after treatment with antisense oligonucleotides in combination with C8 enhancer according to Example 2. **Panel A** shows the treatment with S-PTH-01 antisense oligonucleotide while **Panel B** shows the treatment with S-PTH-02 antisense oligonucleotide. The concentrations of both antisense and enhancers used is indicated. Incubation time was 1 hr for all the samples unless indicated otherwise;
**Fig. 3** shows, as further described in Example 3, the expression of rat PT-1 receptor in intestinal samples of rat treated with antisense oligonucleotide, 25mg C10 or a mix of both. Animals were allowed to recover for 24hrs after treatment;
**Fig. 4** shows, as further described in Example 3, the expression of rat PT-1 receptor in intestinal samples of rat treated with antisense oligonucleotide and 5 or 25mg C10 enhancer. Animals were allowed to recover for 48hrs after treatment; and
**Fig. 5** shows a comparison of transfection efficiencies for β-galactosidase in Caco-2 cells as described in Example 4.

### DETAILED DESCRIPTION OF THE INVENTION

It has now been discovered that permeation enhancers such as C8 and C10 (see later descriptions) can be used to achieve elevated intracellular delivery of antisense oligonucleotides and other nucleic acid-based drugs to the cytoplasm and/or nucleus and other intracellular regions or compartments of epithelial cells, as required for their pharmacological activity. This enhancement in intracellular delivery is unlikely to be restricted to epithelial cells and most likely can be applied to other or all cell types and tissue types. Furthermore, it has been discovered that this enhanced delivery of antisense oligonucleotides upon co-administration with an enhancer can lead to increased biological activity in vitro and in vivo. For instance, administration of an antisense oligonucleotide to a particular receptor in combination with an enhancer can lead to substantially decreased expression of that receptor in cell models and *in vivo* such as in the gastrointestinal tract.

It has further been discovered that enhancers, such as C8 and C10, can also facilitate the intracellular delivery of plasmid DNAs, such as plasmid DNA complexed with a model cationic lipid such as lipofectamine, into Caco-2 cells. For instance, it has been discovered that certain enhancers increase the efficiency of DNA transfection in comparison to DNA complexed with cationic lipids alone. Condensed plasmid DNA complexed with cationic lipids can also be used with the enhancer in order to achieve enhanced intracellular gene delivery as manifested by increased gene expression.

Because of this enhanced intracellular delivery of the oligonucleotides and plasmid DNAs in the presence of various enhancers such as C8 and C10 in epithelial cell models, formulations or drug dosage forms containing these combinations can be used in order to achieve local delivery of nucleic acid-based drugs, such as oligonucleotides or plasmid DNAs, at different sites in the body (be it human or other mammals) including the gastro-intestinal tract, sub-dermal sites, intra-muscular sites, or indeed any sites in the body (human or other mammals) in which both the nucleic acid-based drug and the enhancers can be delivered either together or in sequential fashion. Thus, the nucleic acid-based drug can be administered first followed by administration of the enhancer or the enhancer can be administered first followed by administration of the nucleic acid-based drug. Example routes of administration are oral, parenteral, nasal, inhalation, vaginal, rectal, intradermal and topical. As such, this invention is useful for treating a mammal that can benefit by administration of a pharmacologically effective amount of a nucleic acid-based drug, such as to prevent or treat a condition prevented or ameliorated by the drug.

Furthermore given the ability of enhancers to elevate intracellular delivery of oligonucleotides and plasmid DNAs (either single stranded or double stranded) to mammalian cells, it is proposed that when the enhancers are administered with other nucleic acid-based drugs, such as ribozymes, gene-correcting oligonucleotides, triple-helix forming oligonucleotides, oligonucleotides which function as adjuvants (such as oligonucleotides containing CpG motifs), these nucleic acid-based drug/enhancer combinations will also achieve the improved intracellular delivery of the nucleic acid-based drug into mammalian cells and into tissues.

Additionally, the discovery that enhancers facilitate the intracellular delivery of plasmid DNAs has broad application to the intracellular delivery of plasmid DNAs and genes to mammalian cells using non-viral vector systems including, but not limited to, cationic lipids, PEI (polyethyleneimine) systems, polyanhydride systems, chitosan systems, cellulose systems, dendrimeric based systems, liposomes, PLGA DNA particles co-administered with enhancers. The nucleic acid-based drug can be complexed, for example by covalent or non-covalent bonding, or entrapped by these polymeric systems or can be incorporated as a condensed plasmid complex into such non-viral vector systems using, for example, protamine phosphate or protamine sulphate as the condensing agent.

Additionally, further enhancement of intracellular delivery of a nucleic acid-based drug can be obtained by co-administration of the nucleic acid-based drug/enhancer with an inhibitor of an enzyme that degrades the nucleic acid-based drug, or which expels or effluxes the nucleic acid-based drug from the cell. Example inhibitors of an enzyme that degrades a nucleic acid-based drug include P-glycoprotein inhibitors, such as verapamil, ketaconazole, diltiazem and the like. The nucleic acid-based drug/enhancer combination can also be co-administered with an endosome escape and/or nuclear accumulation agent to further control the location of the nucleic acid-based drug within the cell. Example endosome escape/nuclear accumulation agents include agents having a REDL endosomal escape motif or a KKKRKA nuclear localisation motif. Such agents can be conjugated to agents such as farnesyl, AFCME or myristoyl motifs in order to increase lipophilicity. Additionally, the nucleic acid-based drug can be condensed by a DNA condensing agent, such as polylysine, protamine or calcium phosphate, and administered in the presence of an enhancer.

Furthermore, combinations of oligonucleotides with enhancer type systems may also afford improved intracellular delivery of oligonucleotides into prokaryotic cells including *Escherichia coli, Helicobacter pylori, Salmonella typhininum* and other infectious prokaryotic agents and in doing so facilitate the delivery of nucleic acid-based drugs including oligonucleotides to such infectious agents in order to arrest their growth rate or kill them.

The "enhancer" is preferably a fatty acid or a salt thereof, such as medium chain and long chain fatty acids which is, preferably, solid at room temperature and which, preferably, has a carbon chain length of from 4 to 24 carbon atoms. An "enhancer" also encompasses a combination of one or more enhancers (including physical and covalent/conjugate combination).

Examples of enhancers include, but are not limited to, caprylic acid, nonanoic acid, capric acid, undecanoic acid, lauric acid, acylcarnitines such as palmitoyl carnitine, babassu oil, and fish oils such as eicosapentaenoic acid or docosahexaenoic acid.

Preferably, the enhancer is a medium chain fatty acid, or a salt of a medium chain fatty acid which is, preferably, solid at room temperature and which has a carbon chain length of from 8 to 14 carbon atoms. Preferred medium chain fatty acids for use as enhancers are caprylic, nonanoic, capric, undecanoic and lauric acids. More preferred are caprylic and capric acids.

More preferably, the enhancer is a pharmaceutically acceptable salt, preferably the sodium salt, of a medium chain fatty acid, the medium chain fatty acid having a carbon chain length of from 8 to 14 carbon atoms.

Even more preferably, the enhancer is sodium caprylate, sodium caprate or sodium laurate. As used herein, the terms "C8" and "C10" refer to sodium caprylate and sodium caprate, respectively, and "nonanoate" and "undecanoate" refer to sodium salts.

As used herein, the term "nucleic acid-based drug" means an oligonucleotide, an antisense oligonucleotide including oligonucleotides having a modified backbone chemistry such as phosphorothioate, phosphotriester, methylphosphonate or phosphoramidate oligonucleotides, oligonucleotides having modified sugars or terminal groups such as 2' methoxy substitution, poly-lysine terminally modified oligonucleotides or 2'-O-alkyloligoribonucleotides, chimeric oligonucleotides comprised of nucleotides of different chemistries, oligonucleotides having MOE chemistries or other known chemistries shown to function as antisense oligonucleotides, ribozymes, gene-correcting oligonucleotides, triple-helix forming oligonucleotides, oligonucleotides which function as adjuvants, plasmid DNAs, and genes. In addition a nucleic acid-based drug includes a gene coding for a protein of interest such as a therapeutic protein, a prophylactic or therapeutic vaccine or a cancer chemotherapeutic; a gene coding for an RNA molecule which functions in an antisense capacity when expressed within mammalian cells and a gene coding for a ribozyme.

The amount of enhancer effective to enhance the intracellular delivery of a nucleic acid-based drug ranges from about 0.005mM to about 1M, preferably from about 0.01mM to about 100mM. The optimum concentration for a particular enhancer depends upon a number of factors such as the size of the oligonucleotide to be delivered and the chain length of the enhancer. Particularly preferred concentrations are 1 to 200mM, more preferably 5 to 100mM, for capric acid-derived enhancers and 0.5 to 20mM, more preferably 1 to 10mM for caprylic acid-derived enhancers. Typically, the molar ratio of the enhancer to the nucleic acid-based drug can range from about 1:100 to about 100:1.

Suitable pharmaceutical compositions include liquid, semi-solid or solid dosage forms, such as solutions, suspensions, emulsions, microemulsions, capsules, tablets and microparticulates.

### Example 1 - Uptake of antisense phosphorothioate oligonucleotides in non-differentiated Caco-2 cells in the presence of permeation enhancers.

Caco-2 cells were seeded on coverslips treated with polylysine at a concentration of approximately 2.5 - 5x10⁵ cells/ml and grown to confluence. Cells were washed once with PBS without Ca²⁺, Mg²⁺. 1-2 ml of PBS w/o Ca²⁺, Mg²⁺ containing antisense oligonucleotides +/- enhancers was added and cells were incubated for 30 min at 37°C. Variations to this protocol by pre-incubating cells with enhancers (40min), washing with PBS w/o Ca²⁺, Mg²⁺ and then incubating with antisense oligonucleotides for 30min were also analysed.

Two different FITC labelled antisense oligonucleotides were used: FITC-labelled ELNG05 (5'-GCCCACCGGGTCCACCAT-3'; complementary to a sequence in the human BCR-Abl mRNA) and FITC-labelled S-PTH-05 (5'-CTAAACTTCCCCTCTTGG-3', complementary to sequence 153-170 of the human HPT1 receptor mRNA). The enhancers were used at a range of concentrations: C8 from 120mM to 0.12mM and C10 from 13mM to 0.013mM. The uptake of antisense oligonucleotides was also analysed in the absence of enhancers for variable periods of time (30min - 60min). Following incubation, cells were washed twice with PBS w/o Ca²⁺, Mg²⁺ and stained with 1µg/ml TexasRed-DHPE (dihexadecanoylglycerophosphoethanolamine, Molecular Probe T-1395) for 15min at 37°C, washed extensively and fixed with paraformaldeyde pH shift (Barcallo, 1995) and mounted for analysis by confocal laser scanning microscopy (CLSM).

Variations of this general protocol and results obtained from these experiments are given below.

### Experiment a

Caco-2 cells were incubated with 2ml of enhancers in PBS w/o Ca²⁺ and Mg²⁺ (C8: 80mM and 120mM; C10: 10mM and 13mM) and incubated for 40min at 37°C. After two washes with PBS w/o Ca²⁺ and Mg²⁺, the cells were incubated with 2ml of FITC-antisense oligonucleotide (ELNG05) at a concentration of 10µM in PBS w/o Ca²⁺ and Mg²⁺ and incubated at 37°C for 30min.

Analysis by CLSM indicated that very limited antisense uptake occurred in the absence of any enhancers and that the intracellular distribution was in vesicular compartments. Addition of C8 at 80-120mM and C10 at 10-13mM permeated completely the cells causing intracellular uniform staining with TexasRed-DHPE and FITC-oligonucleotide.

### Experiment b

Caco-2 cells were treated with a PBS w/o Ca²⁺ and Mg²⁺ solution containing both an antisense oligonucleotide and an enhancer (10µM antisense; either S-PTH-05 or ELNG05, C8: 120, 24, 12, 2.4, 1.2, 0.24, 0.12mM; C10: 13, 2.6, 1.3, 0.26, 0.13, 0.026, 0.013mM) and incubated for 30min at 37°C.

CLSM analysis does not show any cytoplasmic or nuclear accumulation of antisense in the absence of enhancers but granular distribution of FITC-staining seems to be present both on the surface and inside the cells consistent with uptake into vesicles but not into nucleus or cytoplasm. Addition of C8 at 120mM confirmed the permeation of the cells and, interestingly, both FITC-oligonucleotide and TexasRed DHPE accumulated in the nucleus. When decreasing the concentration of C8 to 24mM, some cells showed cytoplasmic or nuclear accumulation of FITC-antisense but the majority had a pattern similar to the control. Lower C8 concentrations were identical to the control. The higher concentration of C10 (13mM) caused a loss of adherence of Caco-2 cells in both the experiments. The 2.6 mM C10 concentration did not increase the uptake of antisense.

### Experiment c

Caco-2 cells were incubated with 1ml of antisense +/- enhancers in PBS w/o Ca²⁺ and Mg²⁺ and incubated for 30min at 37°C. Two different fluorescently labelled antisense oligonucleotides were used in these experiments at a concentration of 10µM: ELNG05 (antisense to BCR-Ab1) and S-PTH-05 (antisense to HPT1 receptor). C8 enhancer was used at 120, 100, 80, 60, 40 and 20 mM; C10 enhancer was used at 13, 10, 8, 6, 4 and 2 mM. The uptake of antisense was also analysed in the absence of enhancers for 30min and 60min as control.

In absence of enhancers, granular distribution of the fluorescent-staining was observed; antisense oligonucleotides seem to be present on the surface of the cells and possibly in the cytoplasm after 30min of incubation for both ELNGOS and SPTH-FITC05. With longer incubation (60min), a slight increase of intracellular antisense could be observed.

Using C8 enhancer at a concentration of 120mM, a complete staining of cells with both the fluorescence oligonucleotides used was observed, indicating that cells were completely permeabilized. When the concentration was decreased to 100mM and 60mM, a nuclear accumulation of FITC-antisense was observed. Similar results have been obtained with both the antisense analysed. By decreasing the concentration of C8 further (i.e. 40mM) a decrease uptake was observed and only a few cells show the presence of antisense in vacuoles and inside the plasma membrane.

C10 enhancer caused the destruction of cells at concentrations between 13mM and 8mM. Only a few cells remained attached to the coverslip during the experimental manipulation. At a concentration of 10mM the cells were completely stained, indicating permeabilization of the plasma membrane. At a concentration of 8mM only punctate staining, similar to the control, could be observed.

As shown above, medium chain fatty acid surfactants such as C8 and C10 increase the Caco-2 plasma membrane permeability and promote the uptake of oligonucleotides. Although the highest concentrations of enhancers used in the experiments may cause mortality of cells in culture, but not necessarily cells in tissues, concentrations below the toxicity values are still able to increase the cellular uptake and/or accumulation into the nucleus. Furthermore, prolonged incubation of mammalian cells or tissue with the oligonucleotides and enhancers may further result in enhanced intracellular delivery of the oligonucleotides to mammalian cells or tissues.

### Experiment d - Cytotoxicity of Enhancers on Caco-2 cells

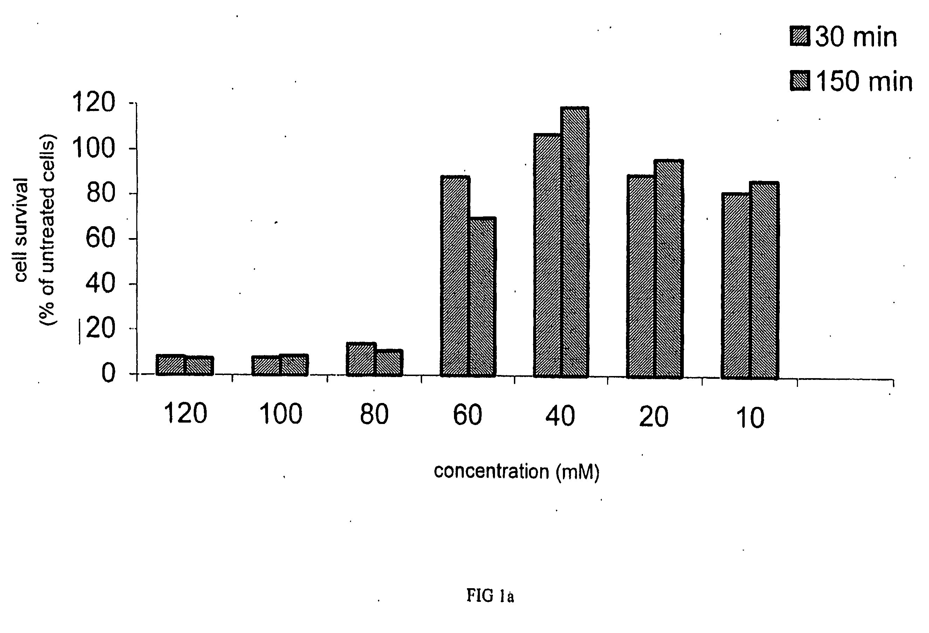

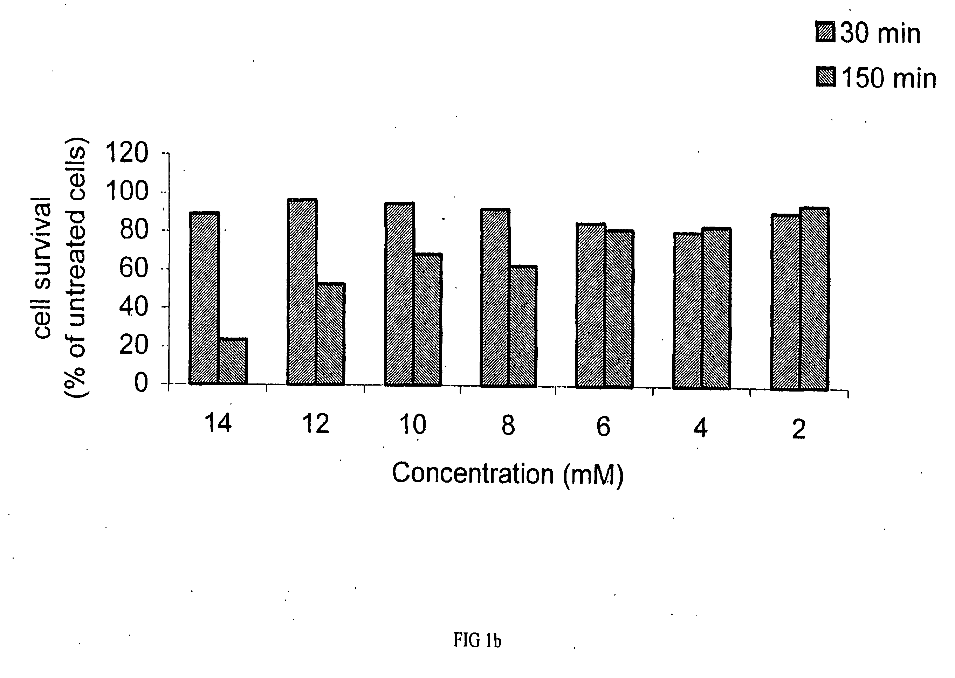

Caco-2 cells were seeded at 10⁵ cells/well in 96-well plates and grown overnight at 37°C. An enhancer at a particular concentration was added to the cells (10µl per well) and cells were incubated for 30min or 150min. 10µl of the MTT labeling reagent from the colorimetric MTT based cell proliferation kit (Boehringer #1465007) was added to each well and cells were incubated for 4 hr at 37°C. 100µl of solubilization buffer was added to each well, cells were incubated overnight and absorbance was read at 570nm. Fig. 1 shows the results of these cytotoxicity studies for C8 (Fig. 1a) and C10 (Fig. 1b) expressed as cell survival (% of untreated cells) for C8 concentrations of 10 to 120 mM and for C10 concentration of 2 to 14 mM.

The above experiments show that the enhancers C8 and C10 dramatically increase the intracellular delivery of FITC-labelled oligonucleotides of the phosphorothioate chemistry. This enhancement in intracellular delivery of the antisense oligonucleotides in the presence of the enhancers C8 and C10 is very rapid, being detectable after 30 minutes in Caco-2 cells. Furthermore, this enhancement in the intracellular delivery of the oligonucleotides is achievable at concentrations of enhancers which are not toxic to Caco-2 cells. From the confocal microscopy studies it is evident that, in the presence of the enhancers, the FITC-labeled oligonucleotides are distributed through-out the cell interior including cytoplasmic and nuclear localisation and are not restricted to punctate distribution which may have been attributed to vesicular or endosomal localisation. More prolonged incubation of mammalian cells with the oligonucleotide/enhancer combination may further enhance the cellular uptake and intracellular distribution to both the cytoplasm and the nucleus. Experiments similar to those described above undertaken with polarised Caco-2 cells gave similar results.

### Example 2 - The effect of antisense oligonucleotides to the HPT1 receptor on the expression of HT-1 in non-differentiated Caco-2 cells: Permeation enhancers promote uptake and biological function of phosphorothioate oligonucleotides.

Caco-2 cells were grown in a 6 well plate until 70-80% confluent. Cells were treated with antisense oligonucleotides S-PTH-Ol (5'-GGGCCTGAAGTATCATAG3'; nucleotides 86-103 of human HPT1 sequence) and S-PTH-02 (5'-CTAAACTTCCCCTCTTGG-3'; nucleotides 153-170 of human HPT1 sequence) at concentrations of 1 or 10 µM in 1ml PBS w/o Ca²⁺ and Mg²⁺. Cellular permeation enhancers C8 and C 10 were added to the antisense mix at concentrations of 40mM, 60mM for C8 and 6mM, 10mM for C10. Caco-2 control wells received either antisense or enhancers only. Untreated cells were also included. Cells were incubated for 30 min or 2h (only a few samples were treated for 2h: 10µM antisense plus 40mM C8 or 6mM C 10), washed twice with PBS and fresh medium was added. Caco-2 cells were incubated for 36h at 37°C.

After incubation, cells were washed twice with PBS and recovered in buffer containing protease inhibitors (0.1M NaCl, 0.01M TrisHCl pH7.6, 1mM EDTA pH8.0, Boehringer Protease Inhibitor cocktail). Samples with floating cells were collected, centrifuged, washed with PBS and resuspended in the same buffer. Cell suspensions were frozen at -80°C. Samples were defrosted, homogenised (hand-homogeniser) and protein concentration quantified by Bradford assay using the Bio-Rad kit. Equal amounts of protein (10µg of total cell lysate) were loaded onto a 10% SDS-polyacrylamide gel for electrophoresis. Once samples were transferred to nitrocellulose membranes, the membranes were subjected to standard Western blot blocking and screening treatments with staining for 1 h with rabbit anti-HPT1 polyclonal antibody (dil. 1:1000) followed by incubation with secondary antibody conjugated to horseradish peroxidase (goat anti rabbit IgG-HRP, Sigma A0545, dil. 1:5000). The results were visualised by enhanced chemiluminescence detection using standard chemiluminescence treatment.

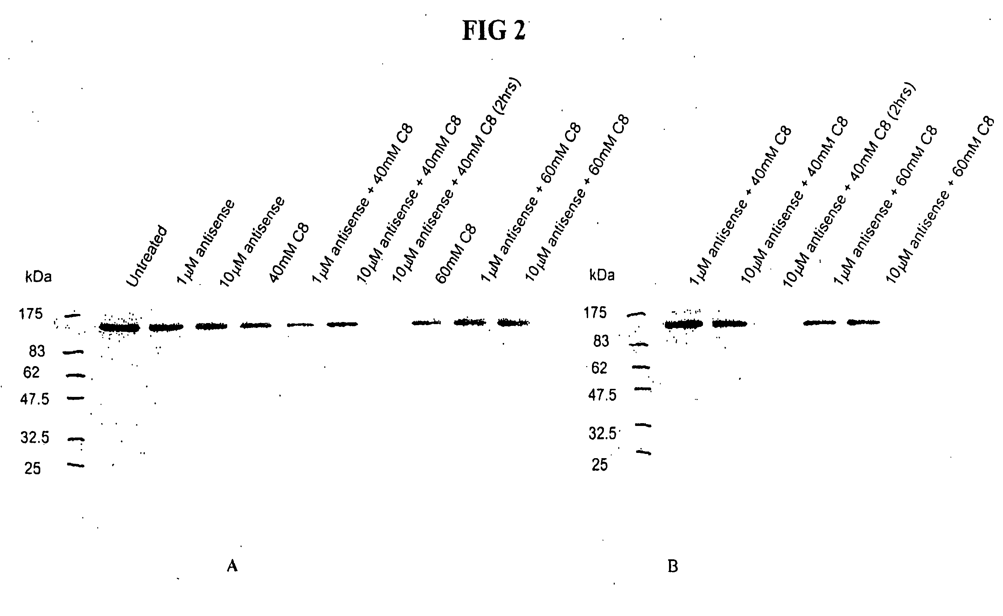

Fig. 2 shows the expression of HPT-1 receptor in Caco-2 non-differentiated cells after treatment with antisense oligonucleotides in combination with C8 enhancer. The treatment with two different antisense oligonucleotides to HPT-1 is indicated. The expression of HPT-1 receptor in non-differentiated Caco-2 cells is substantially decreased after 2hrs incubation with a mix of antisense oligonucleotides and 40mM C8 enhancer.

It is also noteworthy that treatment of the cells with 1µM antisense S-PTH-01 and 40mM C8 for 60 min also resulted in decreased HPT1 expression as determined by Western Blot studies relative to the control, untreated cells (Fig. 2). In addition, treatment of cells with 10µM antisense S-PTH-01 and 60mM C8 for 60 minutes also decreased the expression of HPT1 relative to the control, untreated cells (Fig. 2). Furthermore, treatment of cells with 1 or 10µM S-PTH-02 and 60 mM C8 also decreased the expression of HPT1 relative to untreated cells and relative to cells that were treated with 1µM of this oligonucleotide and 40mM C8 (Fig. 2).

These results indicate that the expression of HPT-1 receptor in non-differentiated Caco-2 cells is substantially decreased after 2hrs incubation with a mix of antisense oligonucleotides and 40mM C8 enhancer.

Furthermore, these results indicate that expression of HPT-1 receptor is decreased in these cells treated with either the S-PTH-01 or S-PTH-02 oligonucleotide together with 40mM or 60mM C8 for 60 minutes relative to control, untreated cells. Thus, a decrease in HPT-1 expression could be argued for lower incubation time (1 hr) for both the concentrations of antisense oligonucleotides used (1 and 10µM).

In Example 1 we demonstrated that treatment of Caco-2 cells with a FITC-labelled oligonucleotide together with either C8 or C 10 results in enhanced intracellular uptake and delivery of the FITC-labelled oligonucleotide to the cells relative to control cells treated with the oligonucleotide alone as determined by confocal microscopy. The studies reported here further demonstrate that treatment of cells with the antisense oligonucleotides S-PTH-01 or S-PTH-02 together with the enhancer C8 at either 40mM or 80mM concentrations for either 60 min or 2 hours results in the down-regulation in expression of the HPT1 receptor as demonstrated by Western Blot studies. Thus we have demonstrated that administration of antisense oligonucleotides to Caco-2 cells together with penetration enhancers increases the intracellular uptake and delivery of the antisense molecules and results in down-regulation of target protein expression. As such, we have demonstrated that co-administration of oligonucleotides together with penetration enhancers increases cellular uptake of same and that such molecules retain activity once delivered to the cells. This is a general way to deliver oligonucleotides to cells and may have application for the delivery of nucleic acids in general to mammalian cells or across biological barriers. In addition, initial treatment of mammalian cells with the enhancer followed by subsequent treatment with the antisense (and vice versa) should also increase the intracellular uptake into mammalian cells and tissue.

### Example 3 - Effect of antisense oligonucleotide to HPT-1 receptor on HPT-1 expression in vivo: The cellular permeation enhancer capric acid (C10) promotes the intracellular uptake and biological function of a phosphorothioate antisense oligonucleotide when delivered in the rat closed loop model.

Wistar rats in the 190-250 g weight range were fasted for 24 hours prior to study initiation. Animals were anaesthetised with sagetal prior to dose administration and during the dosing procedure were given halothane gas. Injections of drug were made directly into the intestine closed loop 12cm below the pyloris and after four hours the sutures were removed from the intestine. The animals were allowed to recover (food and water available) for 24 or 48 hours. At the termination of the experiment the animals were sacrificed and tissue samples from the intestine (site of injection), liver, kidney, spleen, lung and heart were taken, frozen in dry ice and stored at -80°C. Intra-duodenal injection of antisense oligonucleotide alone, C10 enhancer alone or a mixture of antisense oligonucleotide and enhancer were administrated to two groups of animals (N=4 per group) and the rats were allowed to recover for either 24 hour or 48 hours. Antisense oligonucleotide S-PTH-03 (5'-CCACTATAGCTCCCTGAG-3'; antisense to rat PT-1 receptor, nucleotides 119-136) was administered at concentration of 10µM, C10 enhancer was used at 5mg or 25mg in combination with antisense and at 25mg for a control group.

Intestinal samples were defrosted on ice in PBS buffer with protease inhibitors (Boehringer), the intestine was cut with a scalpel and rat intestinal epithelial tissue was prepared by mucosal scraping using a glass slide. The samples were homogenised in a sucrose buffer containing protease inhibitors and the homogenates were centrifuged at 4000 RPM for 15 minutes. 100µg of total protein from each sample were run on 10% SDS-PAGE and blotted onto nitrocellulose membrane. The blot was immunostained with rabbit polyclonal antiserum anti-HPT-1 (rabbit 5, bleed 2, diluted 1:1000) followed by incubation with secondary antibody conjugated to horseradish peroxidase (goat anti rabbit IgG-HRP; Sigma A0545, dil. 1:5000) as outlined previously in the cell studies. The results were analysed by enhanced chemiluminescence detection.

Animal Groups:
1 Antisense only (24 hours)
2 Antisense & C10 5mg (24 hours)
3 Antisense & C10 25 mg (24 hours)
4 C10 25 mg (24 hours)
5 C10 5 mg (48 hours)
6 Antisense & C10 5 mg (48 hours)
7 Antisense & C10 25 mg (48 hours)
8 C10 25 mg (48 hours)
9 Control, untreated

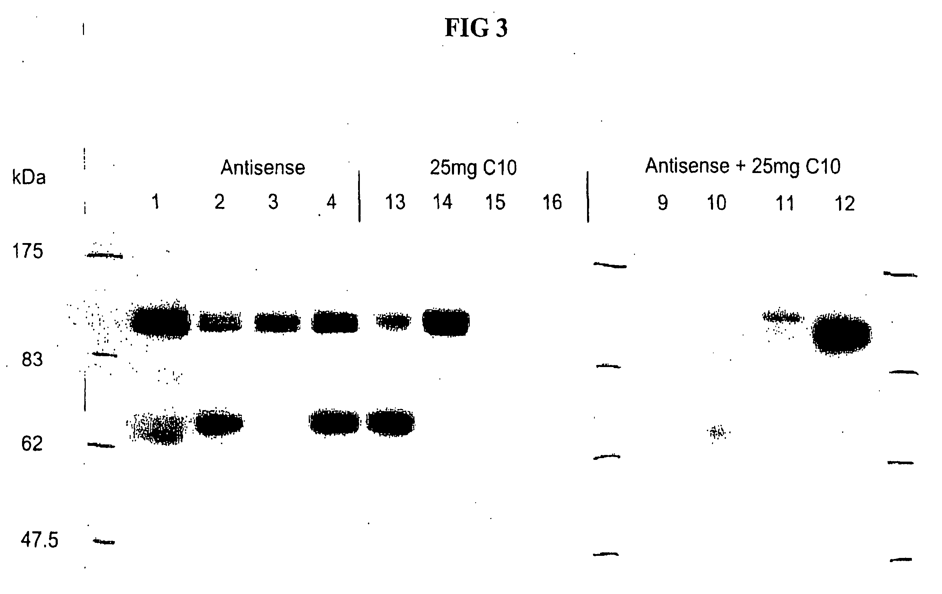

An example of the results obtained is shown in Fig. 3, which shows the expression of rat PT -1 receptor in animals recovered for 24hrs after treatment with antisense oligonucleotide or 25mg C 10 respectively or with a mixture of both. Despite the fact that the level of rat PT-1 expression (120 kDa protein band) is variable from animal to animal within the same treatment group, all four of the rats which were treated with antisense only (i.e. without enhancer) express the protein of interest, whereas only 1 animal treated with the enhancer/antisense combination shows expression of the receptor.

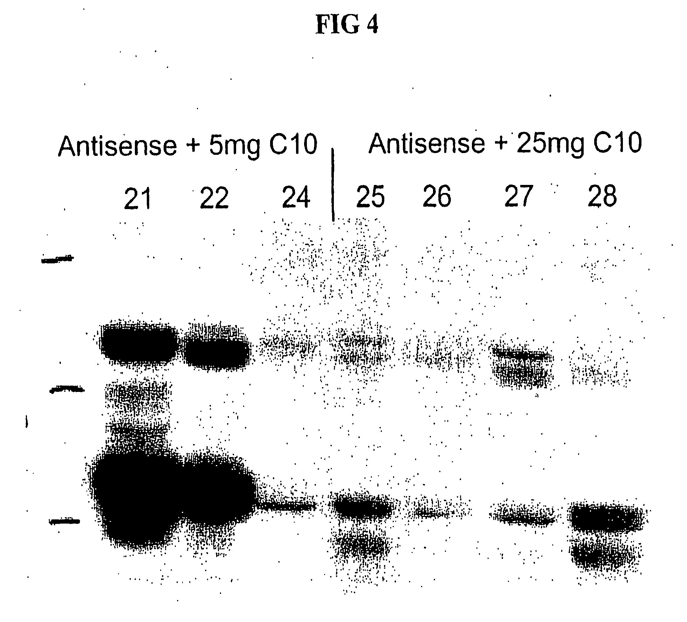

Fig. 4 shows the expression of rat PT-1 receptor in animals recovered for 48hrs after treatment with antisense oligonucleotide in combination with 5mg or 25mg of C10. In this group of rats treated with the lower concentration of C10, 2 rats out of 3 show expression of the protein of interest while the level of expression for the animals treated with 25mg C10 together with the oligonucleotide S-PTH-03 is decreased in all the rats analysed.

Osmolarity and pH values for each formulation injected are given in Table 1 which also provides the weight of the animals both at the beginning and at the end of the study.

This data presented in Figs. 3 and 4 indicate that co-administration of an enhancer plus the antisense oligonucleotide results in decreased expression in the rat PT-1.

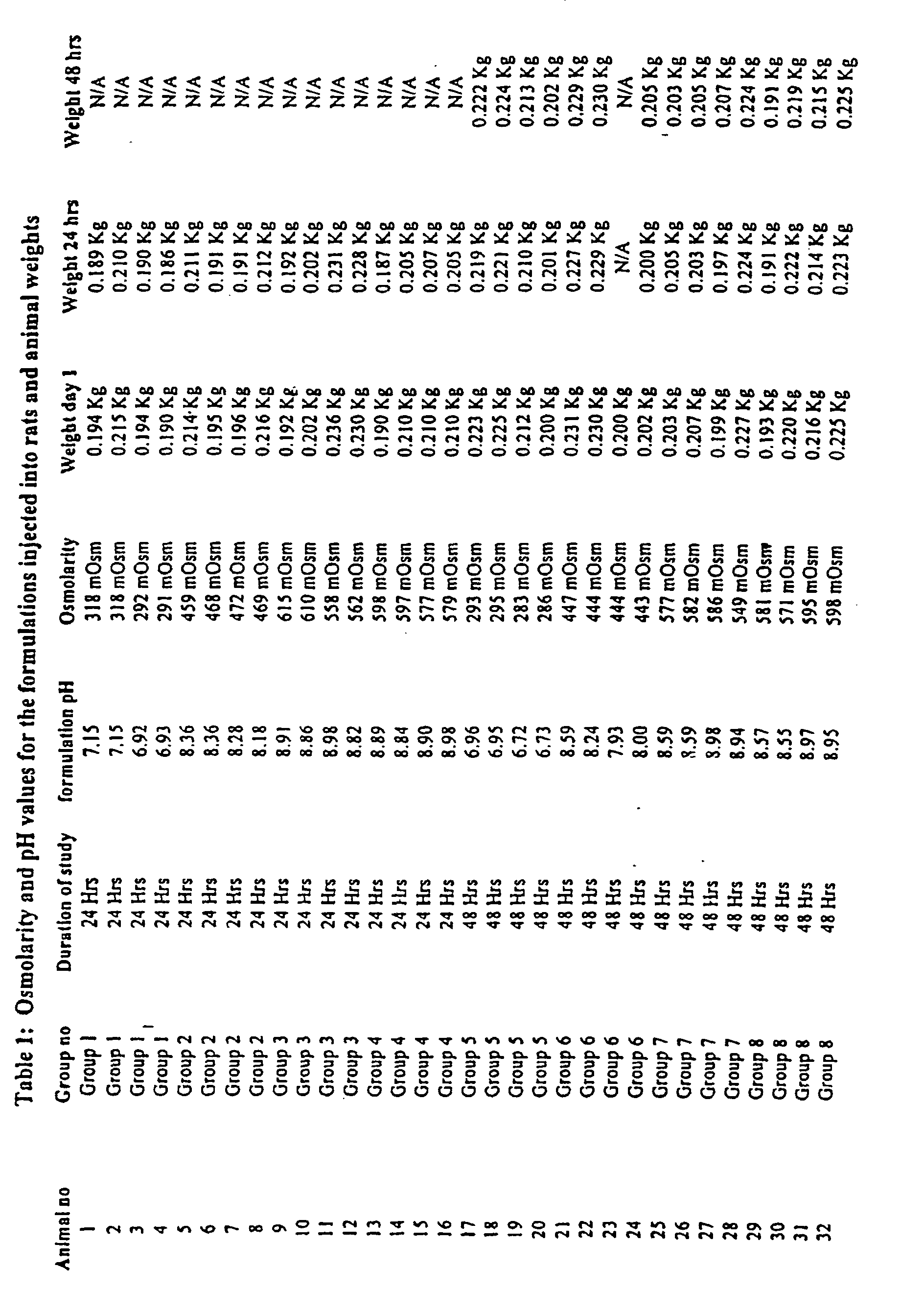

### Example 4 - Effect of co-administration of lipofectamine (cationic lipid) - plasmid DNA (coding for β-galactosidase) complexes with enhancers C8 and C10: Co-administration of the cationic lipid::plasmid DNA complexes increases the transfection of genes into mammalian cells.

A series of transfections examined the effect of the enhancers C8 and C10 on lipofectamine-mediated gene delivery to Caco-2 cells. The DNA used for transfection was a Qiagen maxi-prep of pHM(*lacZ*)6 (0.5µg/µl).

Lipofectamine transfections were accomplished using the following procedure: 3µg (6µl) DNA (solution A) was mixed with 12µl lipofectamine (solution B) [See Table 2 below]. This was a 6X mix. The solution was maintained at room temperature for 15 minutes. The required volume of enhancer stock solution was added [C8 Stock (Na Caprylate): 100mM = 166.2mg in 10ml H₂O; C10 Stock (Na Caprate): 10mM = 19.42mg in 10ml H₂O] followed by addition of Opti-MEM medium up to 600µl. Caco-2 cells grown in the 96-well plate format were washed once with PBS (at 37°C).

The protocol for establishing Caco-2 cells in 96-well tissue-culture plates is as follows: Caco-2 cells were grown in a 30ml 162cm² tissue culture flask to 80% confluency. Cells were suspended in 10ml trypsin-EDTA, centrifuged and resuspended in 5ml complete Caco-2 media. Cell number was determined using a haemocytometer where cells were stained with tryptan blue. 1 x 10⁵ cells were seeded within each well of the 96 well plate (1 x 10⁶ cells were resuspended in 20ml complete Caco-2 media and 200µl aliquots were added to each well of the 96-well plate). Cells were allowed to grow for 24 hours at 37°C with 5% CO₂ prior to transfection.

100µl of DNA mix was added to each of six different wells in the 96-well plates and transfections were left at 37°C (5% CO₂ for 4 hours. The transfection medium was removed and the cells were washed once with PBS (at 37°C). Fresh complete DMEM (100µl) was added to the cells and left O/N at 37°C in the incubator. Cells were assayed for β-galactosidase activity 24 hours post transfection.

**Table 2**

| Trans | Solution A | Solution B | Enhancer vol. |
|---|---|---|---|
| 1 | 6µl DNA + 54µl OptiMEM | 12µl Lipofectamine + 48µl OptiMEM | - |
| 2 | 6µl DNA + 54µl OptiMEM | 12µl Lipofectamine + 48µl OptiMEM | 180 µl C8 (30mM) |
| 3 | 6µl DNA + 54µl OptiMEM | 12µl Lipofectamine + 48µl OptiMEM | 150 µl C10 (5mM) |
| 4 | 6µl DNA + 54µl OptiMEM | 12µl Lipofectamine + 48µl OptiMEM | 30 µl C8 (5mM) |
| 5 | 6µl DNA + 54µl OptiMEM | 12µl Lipofectamine + 48µl OptiMEM | 25 µl C10 (1mM) |

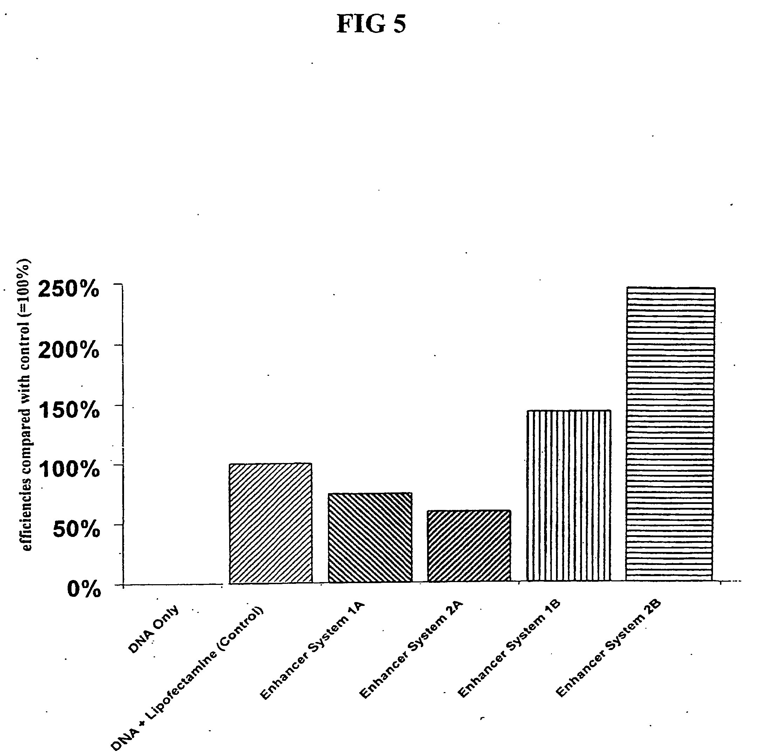

Fig. 5 demonstrates that the penetration enhancers C8 and C10 also facilitate the intracellular delivery of plasmid DNAs into Caco-2 cells when the plasmid DNA is complexed with a model cationic lipid such as lipofectamine. In these studies:
Enhancer system 1A: = 30mM C8
Enhancer system 2A: = 5mM C10
Enhancer system 1B: = 5mM C8
Enhancer system 2B: = 1mM C10

Setting the efficiency of DNA transfection with the cationic lipid: plasmid DNA complex at 100%, in the presence of the enhancers C8 and C10 the efficiency of transfection increases to 143% and 245%, respectively. This demonstration and discovery has general, broad application for the intracellular delivery of plasmid DNAs and genes to mammalian cells using non-viral vector systems such as, by way of example but without limitation, cationic lipids, PEI (polyethyleneimine) systems, polyanhydride systems, chitosan systems, cellulose systems, dendrimeric based systems, liposomes, PLGA DNA particles co-administered with enhancers such as C8 and C10 and systems involving coadministration of a nucleic acid-based drug in conduction with an enhancer.

## Claims

1. Use of an enhancer in the preparation of a medicament containing a nucleic acid-based drug, for the enhancement of intracellular delivery of said nucleic-acid-based drug, **characterised in that**:
the enhancer is a fatty acid or a salt thereof, or an acylcarnitine; and
said intracellular delivery is delivery into the cytoplasm and/or nucleus of a cell;
wherein said enhancement of intracellular delivery is not by topical administration of the medicament.

2. An in vitro method for enhancing the intracellular delivery of a nucleic acid-based drug in mammalian cells comprising contacting the cells with the nucleic acid-based drug and a concentration of an enhancer effective to enhance the intracellular delivery of the nucleic acid-based drug into the cytoplasm or nucleus of a cell, either together or sequentially in either order, wherein:
the enhancer is a fatty acid or a salt thereof or an acylcarnitine; and
wherein said enhancement of intracellular delivery is not by topical administration of the medicament.

3. The method or use of claim 1 or claim 2 wherein the enhancer has a carbon chain length of from 4 to 24 carbon atoms.

4. The method or use of claim 3 wherein the enhancer has a carbon chain length of from 8 to 14 carbon atoms.

5. The method or use of claim 4 wherein the enhancer is caprylic acid, nonanoic acid, capric acid, undecanoic acid, lauric acid or a salt thereof.

6. The method or use of claim 5 wherein the enhancer is caprylic acid, capric acid or a salt thereof.

7. The method or use of claim 6 wherein the enhancer is caprylic acid or a salt thereof.

8. The method or use of any preceding claim wherein the enhancer is a sodium salt of a fatty acid.

9. The method or use of any preceding claim wherein the cell or tissue sample is epithelial.

10. The method or use of any preceding claim wherein the cell or tissue sample is from the gastrointestinal tract.

11. The method or use of claim 10 wherein the cell or tissue sample is of the small intestine.

12. The method or use of any preceding claim, wherein the nucleic acid-based drug is selected from the group consisting of an oligonucleotide, an antisense oligonucleotide, a plasmid DNA, a gene, a ribozyme, a gene-correcting oligonucleotide, a triple-helix forming oligonucleotide, and an oligonucleotide which functions as adjuvant.

13. The method or use of claim 12, wherein the oligonucleotide is selected from the group consisting of an oligonucleotide having a modified backbone chemistry, an oligonucleotide having a modified sugar or terminal group, a chimeric oligonucleotide comprised of nucleotides of different chemistries, and an oligonucleotide having MOE chemistry.

14. The method or use of claim 13, wherein the gene is selected from a gene coding for a protein, a gene coding for an RNA molecule which functions in an antisense capacity when expressed within mammalian cells and a gene coding for a ribozyme.

15. The method or use of any preceding claim, wherein the amount of enhancer effective to enhance the intracellular delivery is about 0.01mM to 1M.

16. The method or use of claim 15, wherein the amount of enhancer effective to enhance the intracellular delivery is about 1mM to 100mM.

17. The method or use of any preceding claim, wherein the molar ratio of the enhancer to the nucleic acid-based drug is 1:100 to 100:1.

18. The use of claim 1, or any one of claims 3 to 17 as dependent from claim 1, wherein the medicament is prepared in a form suitable for oral administration.

19. The method or use of any preceding claim wherein the nucleic acid-based drug is complexed with a cationic lipid.

20. The method or use of any one of claims 1 to 18 wherein the nucleic acid-based drug is complexed with a polymer system.

21. The method or use of any preceding claim wherein the nucleic acid-based drug is entrapped in a polymer system.

22. The method or use of claim 20 or claim 21, wherein the polymer system is selected from the group consisting of a polyethyleneimine system, a polyanhydride system, a chitosan system, a cellulose system, a dendrimeric based system, and PLGA particles.

23. The method or use of any preceding claim wherein an inhibitor of an enzyme that degrades the nucleic acid-based drug or which transports a nucleic acid-based drug back out of the cell is also is also brought into contact with the cell or tissue sample or contained in the medicament.

24. The method or use of claim 23 wherein the inhibitor is a P-glycoprotein inhibitor.

25. The method or use of any preceding claim wherein an endosome escape/nuclear accumulation agent is also brought into contact with the cell or tissue sample, in the method of any preceding claim or contained in the medicament, in the use of any preceding claim.

26. The method or use of any preceding claim wherein the nucleic acid-based drug is condensed by a DNA condensing agent.

27. The method or use of any preceding claim wherein condensed DNA is complexed with cationic lipid and is brought into contact with the cell or tissue sample simultaneously with the enhancer, in the method of any preceding claim or contained in the medicament along with the enhancer, in the use of any preceding claim.

## Patentansprüche

1. Verwendung eines Enhancers bei der Herstellung eines Medikaments, das einen auf Nucleinsäure basierenden Wirkstoff enthält, zur Steigerung intrazellulärer Zufuhr des auf Nucleinsäure basierenden Wirkstoffs, **dadurch gekennzeichnet, dass**:
der Enhancer eine Fettsäure oder ein Salz davon oder ein Acylcarnitin ist; und
die intrazelluläre Zufuhr Zufuhr in das Zytoplasma und/oder den Nucleus einer Zelle ist;
worin die Steigerung der intrazellulären Zufuhr nicht durch topische Verabreichung des Medikaments erfolgt.

2. In-vitro-Verfahren zur Steigerung der intrazellulären Zufuhr eines auf Nucleinsäure basierenden Wirkstoffs in Säugetierzellen, umfassend das Kontaktieren der Zellen mit dem auf Nucleinsäure basierenden Wirkstoff und einer Konzentration eines Enhancers, der wirksam die intrazelluläre Zufuhr des auf Nucleinsäure basierenden Wirkstoffs in das Zytoplasma oder den Nucleus einer Zelle steigert, entweder gleichzeitig oder in beliebiger Reihenfolge nacheinander, worin:
der Enhancer eine Fettsäure oder ein Salz davon oder ein Acylcarnitin ist; und
worin die Steigerung der intrazellulären Zufuhr nicht durch topische Verabreichung des Medikaments erfolgt.

3. Verfahren oder Verwendung nach Anspruch 1 oder Anspruch 2, worin der Enhancer eine Kohlenstoffkettenlänge von 4 bis 24 Kohlenstoffatomen aufweist.

4. Verfahren oder Verwendung nach Anspruch 3, worin der Enhancer eine Kohlenstoffkettenlänge von 8 bis 14 Kohlenstoffatomen aufweist.

5. Verfahren oder Verwendung nach Anspruch 4, worin der Enhancer n-Octansäure, Nonansäure, n-Decansäure, Undecansäure, Dodecansäure oder ein Salz davon ist.

6. Verfahren oder Verwendung nach Anspruch 5, worin der Enhancer n-Octansäure, n-Decansäure oder ein Salz davon ist.

7. Verfahren oder Verwendung nach Anspruch 6, worin der Enhancer n-Octansäure oder ein Salz davon ist.

8. Verfahren oder Verwendung nach einem der vorangehenden Ansprüche, worin der Enhancer ein Natriumsalz einer Fettsäure ist.

9. Verfahren oder Verwendung nach einem der vorangehenden Ansprüche, worin die Zell- oder Gewebeprobe vom Epithel stammt.

10. Verfahren oder Verwendung nach einem der vorangehenden Ansprüche, worin die Zell- oder Gewebeprobe aus dem Magendarmtrakt stammt.

11. Verfahren oder Verwendung nach Anspruch 10, worin die Zell- oder Gewebeprobe aus dem Dünndarm stammt.

12. Verfahren oder Verwendung nach einem der vorangehenden Ansprüche, worin der auf Nucleinsäure basierende Wirkstoff aus der aus einem Oligonucleotid, einem Antisense-Oligonucleotid, einer Plasmid-DNA, einem Gen, einem Ribozym, einem Gen-korrigierendem Oligonucleotid, einem Tripelhelix-bildenden Oligonucleotid und einem Oligonucleotid, das als Adjuvans funktioniert, bestehenden Gruppe ausgewählt ist.

13. Verfahren oder Verwendung nach Anspruch 12, worin das Oligonucleotid aus der aus einem Oligonucleotid mit einer modifizierten Hauptkettenchemie, einem Oligonucleotid mit einer modifizierten Zucker- oder Terminusgruppe, einem Hybrid-Oligonucleotid, das aus Nucleotiden verschiedener chemischer Zusammensetzungen besteht, und einem Oligonucleotid mit MOE-Chemie bestehenden Gruppe ausgewählt ist.

14. Verfahren oder Verwendung nach Anspruch 13, worin das Gen aus einem für ein Protein kodierenden Gen, einem Gen, das für ein RNA-Molekül kodiert, welches mit Antisense-Eigenschaften funktioniert, wenn es innerhalb von Säugetierzellen exprimiert wird, und einem für ein Ribozym kodierenden Gen ausgewählt ist.

15. Verfahren oder Verwendung nach einem der vorangehenden Ansprüche, worin die Enhancer-Menge, die wirksam die intrazelluläre Zufuhr steigert, etwa 0,01 mM bis 1 M beträgt.

16. Verfahren oder Verwendung nach Anspruch 15, worin die Enhancer-Menge, die wirksam die intrazelluläre Zufuhr steigert, etwa 1 mM bis 100 mM beträgt.

17. Verfahren oder Verwendung nach einem der vorangehenden Ansprüche, worin das Molverhältnis zwischen dem Enhancer und dem auf Nucleinsäure basierenden Wirkstoff 1:100 bis 100:1 beträgt.

18. Verwendung nach Anspruch 1 oder einem der Ansprüche 3 bis 17 in Abhängigkeit von Anspruch 1, worin das Medikament zu einer zur oralen Verabreichung geeigneten Form formuliert wird.

19. Verfahren oder Verwendung nach einem der vorangehenden Ansprüche, worin der auf Nucleinsäure basierende Wirkstoff mit einem kationischen Lipid komplexiert wird.

20. Verfahren oder Verwendung nach einem der Ansprüche 1 bis 18, worin der auf Nucleinsäure basierende Wirkstoff mit einem Polymersystem komplexiert wird.

21. Verfahren oder Verwendung nach einem der vorangehenden Ansprüche, worin der auf Nucleinsäure basierende Wirkstoff in ein Polymersystem eingeschlossen wird.

22. Verfahren oder Verwendung nach Anspruch 20 oder Anspruch 21, worin das Polymersystem aus der aus einem Polyethyleniminsystem, einem Polyanhydridsystem, einem Chitosansystem, einem Cellulosesystem, einem System auf Dendrimerbasis und PLGA-Partikeln bestehenden Gruppe ausgewählt ist.

23. Verfahren oder Verwendung nach einem der vorangehenden Ansprüche, worin ein Inhibitor eines Enzyms, das den auf Nucleinsäure basierenden Wirkstoff abbaut oder das einen auf Nucleinsäure basierenden Wirkstoff aus der Zelle zurück transportiert, ebenfalls mit der Zell- oder Gewebeprobe in Kontakt gebracht wird oder im Medikament enthalten ist.

24. Verfahren oder Verwendung nach Anspruch 23, worin der Inhibitor ein P-Glykoprotein-Inhibitor ist.

25. Verfahren oder Verwendung nach einem der vorangehenden Ansprüche, worin ein Endosomaustritts-/Kernakkumulierungs-Mittel im Verfahren nach einem der vorangehenden Ansprüche auch mit der Zell- oder Gewebeprobe in Kontakt gebracht wird oder bei der Verwendung nach einem der vorangehenden Ansprüche im Medikament enthalten ist.

26. Verfahren oder Verwendung nach einem der vorangehenden Ansprüche, worin der auf Nucleinsäure basierende Wirkstoff durch ein DNA-Kondensationsmittel kondensiert wird.

27. Verfahren oder Verwendung nach einem der vorangehenden Ansprüche, worin kondensierte DNA im Verfahren nach einem der vorangehenden Ansprüche mit kationischem Lipid komplexiert wird und gleichzeitig mit dem Enhancer mit der Zell- oder Gewebeprobe in Kontakt gebracht wird oder bei der Verwendung nach einem der vorangehenden Ansprüche im Medikament zusammen mit dem Enhancer enthalten ist.

## Revendications

1. Utilisation d'un activateur dans la préparation d'un médicament contenant un produit à base d'acide nucléique, pour l'activation de la délivrance intracellulaire dudit produit à base d'acide nucléique, **caractérisé en ce que** :
l'activateur est un acide gras ou un sel de celui-ci, ou une acylcarnitine ; et
ladite délivrance intracellulaire est délivrée dans le cytoplasme et/ou le noyau d'une cellule ;
où ladite activation de délivrance intracellulaire n'est pas par administration topique du médicament.

2. Un procédé *in vitro* pour activer la délivrance intracellulaire d'un produit à base d'acide nucléique dans une cellule de mammifère comprenant la mise en contact des cellules avec le produit à base d'acide nucléique et une concentration d'un activateur efficace pour activer la délivrance intracellulaire du produit à base d'acide nucléique dans le cytoplasme ou le noyau d'une cellule, soit en même temps, soit de manière séquentielle dans un ordre quelconque, où :
l'activateur est un acide gras ou un sel de celui -ci, ou une acylcarnitine ; et
où ladite activation de délivrance intracellulaire n'est pas par administration topique du médicament.

3. Le procédé ou l'utilisation de la revendication 1 ou de la revendication 2 où l'activateur a une longueur de chaîne de carbone de 4 à 24 atomes de carbone.

4. Le procédé ou l'utilisation de la revendication 3 où l'activateur a une longueur de chaîne de carbone de 8 à 14 atomes de carbone.

5. Le procédé ou l'utilisation de la revendication 4 où l'activateur est l'acide caprylique, l'acide nonanoïque, l'acide caprique, l'acide undécanoïque , l'acide laurique ou un sel de ces derniers.

6. Le procédé ou l'utilisation de la revendication 5 où l'activateur est l'acide caprylique, l'acide c aprique ou un sel de ces derniers.

7. Le procédé ou l'utilisation de la revendication 6 où l'activateur est l'acide caprylique ou un sel de ce dernier.

8. Le procédé ou l 'utilisation d'une quelconque revendication précédente où l'activateur est un sel de sodium d'un acide gras.

9. Le procédé ou l'utilisation d'une quelconque revendication précédente où la cellule ou l'échantillon de tissu est épithéliale.

10. Le procédé ou l'utilisation d'une quelconque revendication précédente où la cellule ou l'échantillon de tissu est issu du tractus gastro-intestinal.

11. Le procédé ou l'utilisation de la revendication 10 où la cellule ou l'échantillon de tissu provient du petit intestin.

12. Le procédé ou l'utilisation d'une quelconque revendication précédente, où le produit à base d'acide nucléique est choisi d ans le groupe se composant d'un oligonucléotide, d'un oligonucléotide anti-sensibilité, d'un ADN plasmidique, d'un gêne, d'un ribozyme, d'un oligonucléotide correcteur de gêne, d'un oligonucléotique de formation d'hélice triple, et d'un oligonucléotide qui agit en tant qu'adjuvant.

13. Le procédé ou l'utilisation de la revendication 12, où l'oligonucléotide est choisi dans le groupe se composant d'un oligonucléotide ayant une chimie de squelette modifié, d'un oligonucléotide ayant un groupe sucre ou terminal modifié, d'un oligonucléotide chimérique composé de nucléotides de différentes chimie s et d'un oligonucléotide ayant une chimie MOE.

14. Le procédé ou l'utilisation de la revendication 13, où le gêne est choisi parmi un gêne codant pour une protéine, un gêne codant pour une molécule d'ARN qui agit dans une capacité anti-sensibilité lorsqu'il est exprimé à l'intérieur de cellules de mammifère et un gêne codant pour une ribozyme.

15. Le procédé ou l'utilisation d'une quelconque revendication précédente, où la quantité d'activateur efficace pour activer la délivrance intracellulaire est d'environ 0,01mM à 1M.

16. Le procédé ou l'utilisation de la revendication 15, où la quantité d'activateur efficace pour activer la délivrance intracellulaire est d'environ 1mM à 100mN.

17. Le procédé ou l'utilisation d'une quelconque revendication précédente, où le rapport molaire de l'activateur au produit à base d'acide nucléique est de 1:100 à 100:1.

18. L'utilisation de la revendication 1, ou d'une quelconque des revendications 3 à 17 lorsqu'elle dépendent de la revendication 1, où le médicament est préparé dans une forme appropriée pour une administration orale.

19. Le procédé ou l'utilisation d'une quelconque revendication précédente où le produit à base d'acide nucléique est complexé avec un lipide cationique.

20. Le procédé ou l'utilisation d'une quelconque des revendications 1 à 18 où le produit à base d'acide nucléique est complexé avec un système polymère.

21. Le procédé ou l'utilisation d'une quelconque revendication précédente où le produit à base d'acide nucléique est emprisonné dans un système polymère.

22. Le procédé ou l'utilisation de la revendication 20 ou de la revendication 21, où le système polymère est choisi dans le groupe se composant d'un système de polyéthylèneimine, d'un système de polyanhydride, d'un système de chitosane, d'un système de cellulose, d'une système à base de dendrimère, et de particules de PLGA.

23. Le procédé ou l'utilisation d'une quelconque revendication précédente où un inhibiteur d'une enzyme qui dégrade le produit à base d'acide nucléique ou qui transporte un produit à base d'acide nucléique en retour hors de la cellule est également amené en contact avec la cellule ou l'échantillon de tissu, ou est contenu dans le médicament.

24. Le procédé de l'utilisation de la revendication 23 où l'inhibiteur est un inhibiteur de P-glycoprotéïne.

25. Le procédé ou l'utilisation d'une quelconque revendication précédente où un agent d'échappement endosome/d'accumulation nucléaire est également amené en contact avec la cellule ou avec l'échantillon de tissu, dans le procédé d'une quelconque revendication précédente ou est contenu dans le médicament, dans l'utilisation d'une quelconque revendication précédente.

26. Le procédé ou l'utilisation d'une quelconque revendication précédente, où le produit à base d'acide nucléique est condensé par un agent de condensation d'ADN.

27. Le procédé ou l'utilisation d'une quelconque revendication précédente, où l'ADN condensé est complexé avec un lipide cationique et est amené en contact avec la cellule ou avec l'échantillon de tissu de manière simultanée à l'activateur, dans le procédé d'une quelconque revendication précédente ou est contenu dans le médicament en même temps que l'activateur, dans l'utilisation d'une quelconque revendication précédente.
